# EUROPEAN PATENT APPLICATION

(11) **EP 2 589 285 A1**
(43) Date of publication of application: **08.05.2013**
(21) Application number: 11187454.1
(22) Date of filing: 02.11.2011
(51) Int. Cl.: A01H 1/04, A01H 5/08, C12Q 1/68

(54) **Pepino mosaic virus resistant tomato plant**

(71) Applicant: Rijk Zwaan Zaadteelt en Zaadhandel B.V., 2678 KX De Lier (NL)
(72) Inventor: Vogelaar, Arie, 3328 ZC Dordrecht (NL); Gutteling, Evert Willem, 6866 ET Heelsum (NL); Dräger, Dörthe Bettina, 2635 JK Den Haag (NL); Verhoef, Rudolf, 2671 GK Naaldwijk (NL); Van Herwijnen, Zeger Otto, 3043 HB Rotterdam (NL)
(74) Representative: Van Someren, Petronella F. H. M.

(57) **Abstract**

The present invention relates to a tomato plant (*Solanum lycopersicum* L.) comprising a genetic determinant that confers resistance to Pepino Mosaic Virus (PepMV), wherein the resistance is characterised by the presence of at least QTL1, located on Linkage Group (LG) 6 between the physical positions 32363349 bp and 34505939 bp, preferably between positions 33558627 bp and 34505939 bp, or a PepMV-resistance-conferring part thereof, and/or QTL2, located on Linkage Group (LG) 7 between the physical positions 60667821 bp and 62460220 bp, preferably between positions 61387356 bp and 62253846 bp, or a PepMV-resistance-conferring part thereof, and/or QTL3, located on Linkage Group (LG) 9 between the physical positions 60998420 bp and 62512587 bp, preferably between positions 61494664 bp and 62191152 bp, or a PepMV-resistance-conferring part thereof. The invention further relates to seeds and progeny of the plant and to its fruits and processed fruits.

## Description

The present invention relates to a tomato plant (*Solanum lycopersicum* L.) comprising a genetic determinant that confers resistance to Pepino Mosaic Virus (PepMV). The invention further relates to markers and the use of markers for identifying the presence of the genetic determinant that leads to PepMV resistance. The invention also relates to the seeds and progeny of such plants and to propagation material for obtaining such plants. Furthermore the invention relates to the use of the plants, seeds and propagation material that comprises the genetic information as germplasm in a breeding programme.

Commercial vegetable production, including the production of tomato, is affected by many conditions. The choice of the grower for a certain variety is a determining factor, and forms the genetic basis for the result that can be achieved. In addition, there are many external factors that influence the outcome. Growing conditions like climate, soil, and the use of inputs like fertilizer play a major role. In addition to this, the presence of pests and diseases also affect the total yield that can be reached.

Many diseases in tomato have been present for a long time. Breeders have very often identified and incorporated resistances to these diseases from various sources. Examples of this are resistance to *Tobacco Mosaic Virus (TMV), Fusarium oxysporum,* or *Cladosporium fulvum,* and nowadays resistance against those is more or less standard in all commercial tomato varieties. Whenever a new strain or related disease shows up, the search for new sources of resistance has to start all over again. Knowledge of the disease and of the existing resistance to a possibly related form helps to determine new resistance sources relatively quickly. For some diseases however, and especially for pests, it is very difficult or has until now been impossible to develop material with a high level of resistance. Especially when resistance mechanisms are very complex, and rely on several genes that interact with each other, the challenge to develop a good level of resistance can be really high.

In addition, sometimes completely new diseases appear that are not related to any of the already known ones. For these, there is no indication yet what could be the most likely germplasm from which resistance can be developed. Neither is the resistance mechanism known, which also makes the development of a new resistance more complicated. As an additional complicating factor, a good bio-assay is needed to compare resistant plants with susceptible material. When little is known of a new pathogen, first the way in which it can infect a tomato plant has to be determined. A bio-assay that does not correlate with the conditions in the field of a grower could result in contradictory or unsatisfactory results. Too mild or too strong inoculation during a test will not generate useful material to work with for development of a resistant tomato plant in practice. In the end, the ultimate test is whether a resistance holds under a grower's conditions.

In 1999, a new virus occurred in commercial tomato production in Europe, especially in greenhouses. This virus could spread extremely quickly through a whole field, and neighbouring growers were easily affected. The virus was soon identified as Pepino Mosaic Virus, belonging to the Potex Group, which is characterised as highly infectious and persistent.

Pepino Mosaic Virus was first identified in 1974 on pepino or pear melon (*Solanum muricatum*), a South-American crop, on plants originating from Peru. It was at that time determined that tomatoes, and related wild species, could be infected, but without showing symptoms.

It is not yet determined how the virus could suddenly appear in European tomato productions, and later on also in e.g. Canada and the United States. Several different PepMV genotypes are identified and distinguished, among which are: LP, the original one from Peru; EU, from European greenhouses; CH1 and CH2 from Chile; and US1 and US2.

The PepMV isolates that are present in the commercial tomato crops are more virulent in tomato than isolates that are taken from a pepino crop, suggesting that the virus has genetically adapted itself. PepMV spreads very easily mechanically, through the usual activities that are done while working in a tomato crop. Very often therefore infected plants can be seen subsequently in a row. Also tools, clothes, etc. stay capable of transmitting the virus for several weeks, and PepMV can stay in dry plant material for as long as 3 months. It is very difficult to get rid of the virus once it has infected a tomato production.

Symptoms of PepMV are various and largely depend on the plant stage during infection, plant variety, plant vitality, and growing conditions. Sometimes symptoms are hardly visible, but the main symptom expressions include plants with 'nettle heads' - grayish, spiky plant tops -, stunted heads, chlorotic leafs or leaf spots, and uneven ripening, marbling, and blotching of the fruits. Symptoms are most apparent during fall and winter, under low light conditions and lower temperatures.

Losses of tomato production due to PepMV can also vary significantly, depending on the circumstances. In heavily infected crops, losses can probably reach up to 20%.

The presence of other pathogens, for example Verticilium, can strongly influence the yield reduction.

Due to the very easy spread of PepMV, strict hygiene protocols have been implemented in many countries and by many growers. Since it is assumed that PepMV can also be transmitted through infected seeds, hygiene protocols for seed production and seed cleaning are also very strict.

Within the EU, tomato seed has to be free from PepMV when it is imported or traded. EU members are required to do surveys to determine the absence of the virus on tomato seeds.

As it was found to be very difficult to eradicate the virus again from commercial growing, nowadays many growers rely on 'cross-protection': inoculation with a mild isolate to prevent the severe symptoms from aggressive isolates. This system however brings several risks. The combination of certain mild with certain aggressive isolates, especially when they originate from different genotypes, can enhance instead of diminish symptoms (Hanssen et al, Plant Pathology 59, 13-21 (2010)). Since it is not known in advance which aggressive isolate will occur in a certain area or certain season, the possible combination cannot be prevented. In addition, it is not even always clear which mild isolate is being used.

Another risk of combining viral genotypes is the possibility of genetic recombination between the strains, which can result in new and potentially even more devastating virus isolates. (Hanssen et al., European Journal of Plant Pathology 121, 131-146 (2008); Hasi6w-Jaroszewska et al., Acta Biochimica Polonica 57, 385-388 (2010)).

Although the search for sources of resistance to PepMV in tomato has been intensive from the start, until now no resistant *Solanum lycopersicum* plants are available. The genetic background of the resistance, and the bio-assay for screening, are so complex, that no reports or notices of cultivated tomato material with PepMV resistance are known. Durable resistance has only been found in *Solanum ochranthum,* which cannot be crossed with cultivated tomato.

It is an object of the present invention to provide tomato plants (*Solanum lycopersicum* L.) that carry a genetic determinant which leads to resistance to Pepino Mosaic Virus.

It is an object of the present invention to provide QTLs that contribute to Pepino Mosaic Virus resistance in tomato plants (*Solanum lycopersicum*).

It is a further object of the present invention to provide markers that can identify the genetic determinants leading to PepMV resistance.

The present invention thus provides a tomato plant (*Solanum lycopersicum*) comprising a genetic determinant that confers resistance to Pepino Mosaic Virus (PepMV), wherein the resistance is characterised by the presence of at least:
- QTL1 or a PepMV-resistance-conferring part thereof, located on Linkage Group (LG) 6 between the physical positions 32363349 bp and 34505939 bp, preferably between positions 33558627 bp and 34505939 bp, and/or
- QTL2 or a PepMV-resistance-conferring part thereof, located on LG 7 between the physical positions 60667821 bp and 62460220 bp, preferably between positions 61387356 bp and 62253846 bp, and/or
- QTL3 or a PepMV-resistance-conferring part thereof, located on LG 9 between the physical positions 60998420 bp and 62512587 bp, preferably between positions 61494664 bp and 62191152 bp.

The identification of a complex resistance mechanism that requires more than one QTL is a tough and intricate process. No indication of the genetic background for this resistance was known. Furthermore, no good PepMV screening method was publicly available either. An additional complicating factor in the development of the present invention was therefore the challenge to design a good and reliable bio-assay that would generate results that relate well to a grower's conditions.

Research that led to the present invention furthermore showed that the required genetic determinants for PepMV resistance are located on separate chromosomes of the tomato genome. This is an additional complicating factor for the creation of resistant plants.

In one embodiment the PepMV resistance is characterised by the presence of at least:
- QTL1 or a PepMV-resistance-conferring part thereof and QTL2 or a PepMV-resistance-conferring part thereof, or
- QTL1 or a PepMV-resistance-conferring part thereof and QTL3 or a PepMV-resistance-conferring part thereof, or
- QTL2 or a PepMV-resistance-conferring-part thereof and QTL3 or a PepMV_resistance-conferring part thereof.

In one embodiment, the resistance is characterised by the presence of QTL1 or a PepMV-resistance-conferring part thereof, and QTL2 or a PepMV-resistance-conferring part thereof, and QTL3 or a PepMV-resistance-conferring part thereof.

The combination of two or more resistance-conferring QTLs leads to a higher level of resistance to Pepino Mosaic Virus.

In one embodiment, one or more of the genetic determinants that confer resistance to Pepino Mosaic Virus in tomato are present in homozygous form. With respect to the trait of the invention, plants that carry the resistance trait can suitably be identified among descendants from a cross between a plant not carrying the trait, and a plant that does carry the said trait, by growing F2 plants from seeds that are the result from the initial cross and a selfing step, and selecting plants showing the desired trait. Selecting the plants can be done phenotypically through a bio-assay, or can be done through identification of one or more of the QTLs, or a resistance-conferring part thereof, that contribute to the trait.

One or more of the QTLs confer the resistance in a recessive way. The corresponding phenotypic trait, resistance to PepMV, is then consequently also inherited in a recessive way. When all three QTLs confer the resistance in a recessive way, a large population of F2 plants has to be grown to select plants that have the phenotypic trait. Alternatively, selection can start for a lower level of resistance and/or for one or two of the QTLs. The separate genetic determinants conferring the resistance can also be inherited in an intermediate manner, or in a dominant manner. Selection for the phenotypic trait is easier when intermediate or dominant inheritance is involved. A combination of recessive and/or intermediate and/or dominant QTLs can occur.

Selection can be done on phenotype, or on the presence of relevant resistance-conferring QTLs. Selection can also be done by using one or more molecular markers. The use of molecular markers requires a smaller population for screening, and can be done in a very early stage.

In one embodiment, the presence of QTL1 is preferably associated with the molecular marker characterized by SEQ.ID No.1, whereas the presence of QTL2 is preferably associated with the molecular marker characterized by SEQ.ID No.2, and the presence of QTL3 is preferably associated with the molecular marker characterized by SEQ.ID No.3.

It was found according to the invention that QTL1 is located on chromosome 6 of the tomato genome between the physical positions 32363349 bp and 34505939 bp and could be identified by the presence of a molecular SNP marker. This SNP marker is located at 34456931 bp on a physical map and is characterized by SEQ ID No.1 (**Table 2**).

A second QTL contributing to PepMV resistance, QTL2, is positioned on chromosome 7 between the physical positions 60667821 bp and 62460220 bp. QTL2 could be identified by the presence of a SNP marker which is located at a physical position of 61550890 bp, and is characterized by SEQ ID No. 2 (**Table 2**).

The third QTL that leads to PepMV resistance, QTL3, is located on chromosome 9 between the physical positions 60998420 bp and 62512587 bp. The presence of this QTL could be detected by a SNP marker located at 61603006 bp on a physical map and is characterized by SEQ ID No.3 (**Table 2**).

In one embodiment, a tomato plant comprising a genetic determinant that confers resistance to Pepino Mosaic Virus (PepMV), is obtainable by identifying the presence of QTL1 or a PepMV-resistance-conferring part thereof, and/or QTL2 or a PepMV-resistance-conferring part thereof, and/or QTL3 or a PepMV-resistance-conferring part thereof, whereby the QTLs 1 to 3 are defined as:
- QTL1, located on Linkage Group (LG) 6 between the physical positions 32363349 bp and 34505939 bp, preferably between positions 33558627 bp and 34505939 bp;
- QTL2, located on LG 7 between the physical positions 60667821 bp and 62460220 bp, preferably between positions 61387356 bp and 62253846 bp;
- QTL3, located on LG 9 between the physical positions 60998420 bp and 62512587 bp, preferably between positions 61494664 bp and 62191152 bp.

The resistance of the invention is preferably immunity. Immunity is defined as a resistance system wherein the virus particles do not, or not significantly, accumulate in the plant after inoculation or infection. Immunity can be measured through an ELISA assay on virus particles, which is well known to a skilled person. Immunity gives a low or negative score in an ELISA assay. A low or a negative score indicates a virus titer that is comparable to a non-virus-infected plant.

The invention furthermore relates to a cell of a tomato plant as claimed. Such cell may be either in isolated form or may be part of the complete tomato plant or parts thereof and then still constitutes a cell of the invention because such a cell harbours in its genetic constitution the genetic information that leads to the resistance characteristics that define the tomato plant of the invention. Each cell of tomato plants of the invention carries the genetic information that leads to phenotypic expression of said trait. Such a cell of the invention may also be a regenerable cell that can be used to regenerate a new tomato plant of the invention.

The invention also relates to tissue of a plant as claimed. The tissue can be undifferentiated tissue or already differentiated tissue. Undifferentiated tissues are for example stem tips, anthers, petals, pollen and can be used in micropropagation to obtain new plantlets that are grown into new plants of the invention. The tissue can also be grown from a cell of the invention.

The invention according to a further aspect thereof relates to seeds of a plant as claimed. Although the seeds do not show the genetic trait of the tomato plant of the invention, they harbour the genetic information that when a plant is grown from the seeds makes this plant a plant of the invention.

The invention also relates to progeny of the plants, cells, tissues and seeds of the invention. Such progeny can in itself be plants, cells, tissues or seeds.

As used herein the word "progeny" is intended to mean the first and all further descendants from a cross with a plant of the invention that comprises a genetic determinant that leads to PepMV resistance. Progeny of the invention are descendants of any cross with a plant of the invention that carries the trait that leads to PepMV resistance.

"Progeny" also encompasses plants that carry the resistance trait of the invention and are obtained from other plants or progeny of plants of the invention by vegetative propagation or multiplication.

The invention thus further relates to seed of the claimed plant and to parts of the plant that are suitable for sexual reproduction. Such parts are for example selected from the group consisting of microspores, pollen, ovaries, ovules, embryo sacs and egg cells. In addition, the invention relates to parts of the plant that are suitable for vegetative reproduction, in particular cuttings, roots, stems, cells, and protoplasts.

According to a further aspect thereof the invention provides a tissue culture of the claimed plant. The tissue culture comprises regenerable cells. Such tissue culture can be derived from leaves, pollen, embryos, cotyledons, hypocotyls, meristematic cells, roots, root tips, anthers, flowers, seeds and stems.

The invention furthermore relates to hybrid seed and to a method of producing hybrid seed comprising crossing a first parent plant with a second parent plant and harvesting the resultant hybrid seed, wherein said first parent plant and/or said second parent plant is the plant as claimed.

The invention also relates to inbreds and doubled haploids.

In one embodiment, the invention relates to tomato plants of the invention that carry the genetic determinant which leads to PepMV resistance, and having acquired said determinant by introduction of the genetic information that is responsible for the trait from a suitable source, either by conventional breeding, or genetic modification, in particular by cisgenesis or transgenesis. Cisgenesis is genetic modification of plants with a natural gene, coding for an (agricultural) trait, from the crop plant itself or from a sexually compatible donor plant. Transgenesis is genetic modification of a plant with a gene from a non-crossable species or a synthetic gene.

The invention also relates to the germplasm of plants of the invention. The germplasm is constituted by all inherited characteristics of an organism and according to the invention encompasses at least the trait of the invention. The germplasm can be used in a breeding programme for the development of PepMV resistant tomato plants.

The invention also relates to a tomato fruit that is produced by a plant of the invention. The invention further relates to a food product, comprising the fruit of a tomato plant as claimed, or parts thereof. The invention also relates to a food product in processed form.

The term 'genetic determinant' as used herein encompasses one or more QTLs, genes, or alleles. These terms are used interchangeably. 'Genetic background' or 'genetic information' can be used instead of 'genetic determinant', and can comprise more than one relevant QTL, gene, or allele. In addition the phrase 'genetic determinants' can be used. 'Genetic background' can also include more of the genome than only the QTL, gene, or allele that is indicated or that is identified. Genetic background can in addition be defined as 'genotype'.

A genetic determinant can be identified by the use of a molecular marker. A genetic determinant can alternatively be identified by the position on a genetic map, or by indication of the location on a linkage group or chromosome. When a genetic determinant is not linked to a specific molecular marker any longer, but its position on a chromosome as defined on a genetic map is unaltered, this genetic determinant is still the same as when it was linked to the molecular marker. The genetic trait that it confers is therefore also still the same.

The 'genetic trait' is the trait or characteristic that is conferred by the genetic determinant. The genetic trait can be identified phenotypically, for example by performing a bio-assay. However, also plant stages for which no phenotypic assay can be performed do carry the genetic information that leads to the genetic trait. 'Trait' or 'phenotypic trait' can be used instead of 'genetic trait'.

In the absence of molecular markers, equivalence of genetic determinants can be determined by an allelism test. To perform an allelism test, material that is homozygous for the known determinant is crossed with material that is homozygous for the genetic determinant to be tested. When no segregation for the trait to be observed is present in the F2 of the cross, the genetic determinants have been proven to be the same.

When more than one gene is responsible for a certain trait, and an allelism test is done to determine equivalence, the skilled person doing the test has to make sure that all relevant genes are present homozygously for the test to work properly.

Genetic maps can vary according to the method by which they are assembled. A person skilled in the art knows how to compare and combine genetic maps, whereby differences between genetic maps can be eliminated or minimized. Information from one genetic map can therefore be transferred or translated to another genetic map. The positions as used herein are physical positions based on the public physical map of the tomato genome, release SL2.40 of January 2011 (http://solgenomics.net/genomes/ Solanum_lycopersicum/genome_data.pl).

Resistance to Pepino Mosaic Virus based on the genetic determinant of the invention comprises resistance to one or more known PepMV genotypes. Known genotypes include LP, EU, CH1, CH2, US1 and US2. Resistance to Pepino Mosaic Virus can in addition comprise resistance to a PepMV genotype that has currently not yet been identified. A not yet identified PepMV genotype can be a genotype that is presently infecting plants, but has not yet been characterised as belonging to Pepino Mosaic Virus. It can also be a new PepMV genotype that has developed from existing strains, or another genotype that will also be classified as being Pepino Mosaic Virus.

The invention will be further illustrated in the Examples that follow.

### EXAMPLES

### EXAMPLE 1

### Creation of tomato plants of the invention

In research that led to the present invention, a highly resistant plant was identified in the applicant's germplasm by means of performing bio-assays (see Example 2) for Pepino Mosaic Virus resistance. The resistance was repeatedly confirmed by consecutive bio-assays.

The self-incompatible resistant source was crossed with a *Solanum lycopersicum* line. Embryo rescue was performed to obtain F1 offspring of this interspecific cross. This F1 was again self-incompatible, and intercrossing of various F1 plants was carried out to obtain an F2 population.

A large number of F2 plants was tested in replicate, by making 10 cuttings of each plant to obtain identical genotypes. The PepMV resistance test identified only two highly resistant F2 plants. The highly resistant plants were used for further backcrossing, as known by the skilled person. After two backcrossing and selfing steps, a bio-assay was performed to identify highly resistant plants and to confirm the high level of resistance. The plants thus obtained are further used as the source of the resistance.

### EXAMPLE 2

### Bio-assay for Pepino Mosaic Virus resistance

Pepino Mosaic Virus includes very aggressive genotypes, but its behaviour and symptom exhibition can be unpredictable. It is therefore extremely important for the development of resistant plants to perform a very thorough assay with sufficient repetitions to ensure a good performance in growers' conditions.

From the plants to be tested, several cuttings are made to do the assay on several plants of the same genetic background. This approach prevents the selection of 'escapes', i.e. plants that are for some reason identified as being resistant, but just due to the circumstances and not because they contain genetic determinants that lead to resistance.

Mechanical inoculation is done by a standard method as is known in the art, starting with a low virus titer with a 300 times dilution. The plants that show phenotypic resistance to PepMV are selected. They comprise one or more genetic determinants for PepMV resistance.

Cuttings are made from the selected plants to start again with a young plant stage. The selected plants are re-inoculated with a high virus titer, i.e. a 30 times dilution. In this way, the strongest plants containing the best genetic determinants are identified.

In addition to the bio-assay, an ELISA assay is performed to determine that the plants are not just symptomless, but that the virus is also not multiplied in the plants. Plants with a score <0.1, which have an absorption lower than 0.1 in the ELISA test, are confirmed as resistant plants. These plants have an absorption similar to non-inoculated controls. Plants with an absorption lower than 0.1, which is an absorption similar to non-inoculated controls, have a resistance mechanism that is characterised as immunity.

Resistant plants are selected and backcrossed to start the next cycle. Symptom observation and ELISA testing is continued during the growing period, to ensure that the plants have a durable resistance.

**Table 1**

| Segregation for PepMV resistance of selected BC2S1 families | | | | | | |
|---|---|---|---|---|---|---|
| | **# of plants with absorption:** | | | | | |
| **BC2S1 family** | **< 0,1** | **> 0,1 & < 1,0** | **> 1,0** | **NA** | **total** | **% R** |
| 6278-8 | 22 | 3 | 120 | 5 | 150 | 14,7 |
| 6277-2 | 34 | 2 | 105 | 0 | 141 | 24,1 |
| 6277-3 | 60 | 2 | 79 | 0 | 141 | 42,6 |
| 6277-16 | 28 | 8 | 106 | 0 | 142 | 19,7 |
| 6277-25 | 35 | 1 | 103 | 3 | 142 | 24,6 |
| 6278-3 | 39 | 18 | 89 | 4 | 150 | 26,0 |
| 6282-3 | 27 | 1 | 109 | 0 | 137 | 19,7 |
| 6282-4 | 28 | 1 | 107 | 1 | 137 | 20,4 |
| 6282-10 | 24 | 1 | 109 | 1 | 135 | 17,8 |
| 6282-11 | 20 | 0 | 114 | 1 | 135 | 14,8 |
| 6284-7 | 23 | 4 | 104 | 0 | 131 | 17,6 |
| 6284-12 | 54 | 5 | 87 | 2 | 148 | 36,5 |

From the table it follows that the selected BC2S1 families segregate for immune (<0.1) and susceptible plants (>1.0).

### EXAMPLE 3

### QTL mapping and marker development

A large population of BC1S1 plants as obtained from Example 1, were genotyped with 880 SNP markers. 281 of these SNP markers were polymorphic and were used to do a QTL analysis for the PepMV resistance. Remarkably, 3 QTLs that contributed to the resistance were located, positioned on 3 separate chromosomes. QTL SNP markers that correlated most closely to the QTLs are presented in **Table 2**.

**Table 2**

| Genetic SNP markers that are able to identify the presence of QTLs involved in PepMV resistance in *Solanum lycopersicum* | | | |
|---|---|---|---|
| SNP sequence | | | Position bp |
| QTL1 | SEQ ID No.1 | GATGATCCCCCAATG[A/G]TCAAGAAATCTTGCA | 34456931 |
| | | | |
| QTL2 | SEQ ID No.2 | CACTGGTGAAAAAGT[C/G]GCAATTAAAAAAATT | 61550890 |
| | | | |
| QTL3 | SEQ ID No.3 | CTCTCAAGTTCCAGA[C/T]ACCGCTTCTGAGGGA | 61603006 |

## Claims

1. Tomato plant (*Solanum lycopersicum* L.) comprising a genetic determinant that confers resistance to Pepino Mosaic Virus (PepMV), wherein the resistance is **characterised by** the presence of at least:
- QTL1, located on Linkage Group (LG) 6 between the physical positions 32363349 bp and 34505939 bp, preferably between positions 33558627 bp and 34505939 bp, or a PepMV-resistance-conferring part thereof, and/or
- QTL2, located on Linkage Group (LG) 7 between the physical positions 60667821 bp and 62460220 bp, preferably between positions 61387356 bp and 62253846 bp, or a PepMV-resistance-conferring part thereof, and/or
- QTL3, located on Linkage Group (LG) 9 between the physical positions 60998420 bp and 62512587 bp, preferably between positions 61494664 bp and 62191152 bp, or a PepMV-resistance-conferring part thereof.

2. Tomato plant as claimed in claim 1, wherein the resistance is **characterised by** the presence of at least:
- QTL1 or a PepMV-resistance-conferring part thereof and QTL2 or a PepMV-resistance-conferring part thereof, or
- QTL1 or a PepMV-resistance-conferring part thereof and QTL3 or a PepMV-resistance-conferring part thereof, or
- QTL2 or a PepMV-resistance-conferring-part thereof and QTL3 or a PepMV_resistance-conferring part thereof.

3. Tomato plant as claimed in claim 1 or 2, wherein the resistance is **characterised by** the presence of QTL1 or a PepMV-resistance-conferring part thereof, and QTL2 or a PepMV-resistance-conferring part thereof, and QTL3 or a PepMV-resistance-conferring part thereof.

4. Seed of a tomato plant as claimed in any one of the claims 1-3, wherein the plant that can be grown from the seed comprises one or more of the QTLs 1 to 3 as defined in claim 1.

5. Seed as claimed in claim 4, wherein the plant that can be grown from the seed is resistant to Pepino Mosaic Virus.

6. Progeny of a tomato plant as claimed in any one of the claims 1-3 or of tomato seed as claimed in claim 4 or 5, comprising one or more of the QTLs 1 to 3 as defined in claim 1.

7. Progeny as claimed in claim 6, which is resistant to Pepino Mosaic Virus.

8. Propagation material suitable for producing a plant as claimed in any one of the claims 1-3, 6 and 7 or seed as claimed in claim 4 or 5.

9. Propagation material as claimed in claim 8, which propagation material is formed by parts of the plant that are suitable for sexual reproduction, in particular microspores, pollen, ovaries, ovules, embryo sacs and egg cells.

10. Propagation material as claimed in claim 8, which propagation material is formed by parts of the plant that are suitable for vegetative reproduction, in particular cuttings, roots, stems, cells and protoplasts, tissue cultures of regenerable cells.

11. Propagation material as claimed in claim 10,
wherein the parts of the plant suitable for tissue culture comprise leaves, pollen, embryos, cotyledons, hypocotyls, meristematic cells, roots, root tips, microspores, anthers, flowers, seeds and stems.

12. Propagation material as claimed in any one of the claims 8 to 11, wherein the plant produced from the propagation material comprises one or more of the QTLs 1 to 3 as defined in claim 1.

13. Propagation material as claimed in any one of the claims 8 to 12, wherein the plant produced from the propagation material is resistant to Pepino Mosaic Virus.

14. Tomato plant as claimed in any of the claims 1 to 3, 6 and 7, seeds as claimed in claim 4 or 5 or propagation material as claimed in any one of the claims 8 to 12, wherein:
- the presence of QTL1 can be identified by a molecular marker **characterized by** SEQ.ID No.1,
- the presence of QTL2 can be identified by a molecular marker **characterized by** SEQ.ID No.2,
- the presence of QTL3 can be identified by a molecular marker **characterized by** SEQ.ID No.3.

15. A tomato fruit of a plant as claimed in any one of the claims 1 to 3, 6 and 7.

16. Food product, comprising the tomato fruit as claimed in claim 15, or parts thereof.

17. Food product as claimed in claim 16 in processed form.

18. Use of a plant as claimed in any one of the claims 1 to 3, 6 and 7 or plants produced from the seeds of claim 4 or 5 or from the propagation materials as claimed in any one of the claims 8 to 12 as germplasm in a breeding programme for the development of Pepino Mosaic Virus resistant tomato plants.
